# EUROPEAN PATENT APPLICATION

(11) **EP 4 400 573 A1**
(43) Date of publication of application: **17.07.2024**
(21) Application number: 22867274.7
(22) Date of filing: 01.09.2022
(51) Int. Cl.: C12J 1/00, A23L 27/00

(54) **ACETIC-ACID-CONTAINING POWDER**

(30) Priority: 09.09.2021 JP 2021146568
(71) Applicant: Fuso Chemical Co., Ltd., Osaka-shi, Osaka 541-0041 (JP)
(72) Inventor: MATSUO, Midori, Osaka-shi, Osaka 541-0041 (JP); SAKAUE, Narumi, Osaka-shi, Osaka 541-0041 (JP); OKAYAMA, Tatsuya, Osaka-shi, Osaka 541-0041 (JP)
(74) Representative: Müller-Boré & Partner Patentanwälte PartG mbB
(86) International application number: PCT/JP2022/032899
(87) International publication number: WO 2023/037952

(57) **Abstract**

Disclosed is an acetic-acid-containing powder comprising acetic acid or a liquid containing acetic acid, and a substrate, wherein the acetic acid or the liquid containing acetic acid is adsorbed on the substrate, the substrate comprising at least one member selected from the group consisting of malic acid salts, gluconic acid salts, tartaric acid salts, and lactic acid salts. This acetic-acid-containing powder has excellent taste and can be easily produced.

## Description

### Technical Field

The present invention relates to an acetic-acid-containing powder.

### Background Art

Acetic acid cannot be present in powder form at ordinary temperature and ordinary pressure. Since acetic acid is highly volatile, various difficulties are involved in producing an acetic-acid-containing powder.

As a method for producing an acetic-acid-containing powder, for example, a method for producing a brewed vinegar powder has been proposed, which comprises obtaining concentrated brewed vinegar by removing ice crystals produced by freezing brewed vinegar, and allowing the resulting concentrated brewed vinegar to be adsorbed on sodium acetate powder (Patent Literature (PTL) 1). Another method for producing a brewed vinegar powder has also been proposed, which comprises mixing and dissolving brewed vinegar, dextrin with a DE of 6 to 13, and a predetermined electrolyte to prepare an undiluted solution having an acetic acidity of 1 to 25 wt%, and spray-drying the undiluted solution while keeping the temperature at 20 to 60°C (PTL 2).

### Citation List

### Patent Literature

PTL 1: JPS41-16117B
PTL 2: JPS48-26991A

### Summary of Invention

### Technical Problem

The present inventors found that the powder obtained by allowing brewed vinegar to be adsorbed on sodium acetate as in PTL 1 has problems, such as having bitter and astringent taste specific to sodium acetate, which is undesirable in terms of taste when adding it to foods. Further, the present inventors found the following problems in forming brewed vinegar into a powder using dextrin as a substrate as in PTL 2. That is, for example, an apparatus specific to spray-drying is required, which results in high costs for installing equipment. Additionally, the acetic acid content in the resulting powder is low since acetic acid is released during the heat-drying process (for example, for the purpose of lowering the pH, the amount of powder for use will increase). Furthermore, only slight moisture absorption causes, for example, deliquescence and solidification.

A main problem of the present invention is to provide an acetic-acid-containing powder that has excellent taste and that can be easily produced.

### Solution to Problem

The present inventors conducted extensive research to solve the above problem and found that an acetic-acid-containing powder comprising as a substrate at least one member selected from the group consisting of malic acid salts, gluconic acid salts, tartaric acid salts, and lactic acid salts can suppress bitter and astringent taste etc. and is thus excellent in terms of taste, and can be easily produced without using equipment for spray-drying. The present invention has been completed through further studies based on such findings.

The present invention encompasses the following embodiments.

### Item 1.

An acetic-acid-containing powder comprising acetic acid or a liquid containing acetic acid, and a substrate, wherein the acetic acid or the liquid containing acetic acid is adsorbed on the substrate, the substrate comprising at least one member selected from the group consisting of malic acid salts, gluconic acid salts, tartaric acid salts, and lactic acid salts.

### Item 2.

The acetic-acid-containing powder according to Item 1, wherein the substrate comprises at least one member selected from the group consisting of sodium malate, potassium malate, sodium gluconate, potassium gluconate, calcium gluconate, potassium tartrate, and calcium lactate.

### Advantageous Effects of Invention

The present invention provides an acetic-acid-containing powder that has excellent taste and that can be easily produced.

### Description of Embodiments

The acetic-acid-containing powder of the present invention is a powder in which acetic acid or a liquid containing acetic acid is adsorbed (or immobilized) on a specific substrate.

In the present specification, the term "acetic acid" is used to include glacial acetic acid. The liquid containing acetic acid may be, for example, a diluted solution of acetic acid (e.g., a solution diluted with water), vinegar (table vinegar), or the like. Of these, vinegar (table vinegar) is preferred. In the case of vinegar (table vinegar), the acetic-acid-containing powder may be referred to as a "vinegar powder." Table vinegar is broadly divided into brewed vinegar and synthetic vinegar.

Examples of brewed vinegar include the following (A1) to (A6):
(A1) a liquid seasoning obtained by acetic acid fermentation of mash obtained by using, as a raw material, agricultural products, such as grains, fruits, and vegetables, processed products thereof (e.g., squeezed juice), or honey;
(A2) a liquid seasoning obtained by acetic acid fermentation of a mixture obtained by adding an alcohol or sugars to the mash mentioned above;
(A3) a liquid seasoning obtained by acetic acid fermentation of an alcohol;
(A4) a liquid seasoning obtained by acetic acid fermentation of a mixture obtained by adding an agricultural product, a processed product thereof, or honey to an alcohol;
(A5) a mixed liquid seasoning of two or more members selected from the group consisting of (A1) to (A4); and
(A6) a mixture obtained by adding sugars, acidulants other than acetic acid, seasonings, such as amino acids, table salt, etc. to the mixed liquid seasoning.

Representative examples of brewed vinegar include grain vinegar, fruit vinegar, and vegetable vinegar. Examples of grain vinegar include rice vinegar, rice black vinegar, brown rice vinegar, brown rice black vinegar, barley black vinegar, adlay vinegar, and wheat vinegar. Examples of fruit vinegar include apple vinegar, grape vinegar, persimmon vinegar, and plum vinegar. Examples of vegetable vinegar include tomato vinegar.

Examples of synthetic vinegar include the following (B1) and (B2):
(B1) a liquid seasoning obtained by adding sugars, acidulants other than acetic acid, seasonings, such as amino acids, table salt, etc. to a diluted acetic acid solution; and
(B2) a mixture obtained by adding brewed vinegar to a diluted acetic acid solution.

Vinegar (table vinegar) may be used as is or after concentration. Examples of concentration methods include, but are not particularly limited to, a method of removing ice crystals formed by freezing vinegar.

The acidity of the liquid containing acetic acid may be, for example, 1% or more, 2% or more, 3% or more, 4% or more, or 5% or more, and may be 99% or less, 95% or less, 90% or less, 85% or less, 80% or less, 75% or less, 70% or less, 65% or less, 60% or less, 55% or less, or 50% or less. The acidity can be determined, for example, by HPLC or neutralization titration.

The present invention is characterized by using a substrate comprising at least one member selected from the group consisting of malic acid salts, gluconic acid salts, tartaric acid salts, and lactic acid salts, as a substrate capable of adsorbing acetic acid or the liquid containing acetic acid. The use of such a substrate can suppress bitter and astringent taste etc., and thus the taste significantly improves.

The salts are not particularly limited and may be, for example, metal salts. Examples of metal salts include alkali metal salts and alkaline earth metal salts. Examples of alkali metal salts include sodium salts and potassium salts. Examples of alkaline earth metal salts include magnesium salts and calcium salts. In one embodiment, the salts are preferably hydrates (hydrated salts).

In one embodiment, the substrate preferably comprises or consists of at least one member selected from the group consisting of sodium malate, potassium malate, sodium gluconate, potassium gluconate, calcium gluconate, potassium tartrate, and calcium lactate, more preferably comprises or consists of at least one member selected from the group consisting of sodium malate, potassium malate, potassium gluconate, calcium gluconate, potassium tartrate, and calcium lactate, and still more preferably comprises or consists of at least one member selected from the group consisting of sodium malate, potassium malate, potassium gluconate, and calcium gluconate.

In another embodiment, the substrate preferably comprises or consists of a malic acid salt and/or a gluconic acid salt. In this embodiment, the substrate preferably comprises or consists of a malic acid salt, more preferably comprises or consists of an alkali metal salt of malic acid, still more preferably comprises or consists of at least one member selected from the group consisting of sodium malate and potassium malate, and even more preferably comprises or consists of sodium malate. Alternatively, the substrate preferably comprises or consists of a gluconic acid salt, more preferably comprises or consists of at least one member selected from the group consisting of an alkali metal salt of gluconic acid and an alkaline earth metal salt of gluconic acid, still more preferably comprises or consists of at least one member selected from the group consisting of sodium gluconate, potassium gluconate, and calcium gluconate, and even more preferably comprises or consists of potassium gluconate.

In another embodiment, the substrate preferably comprises or consists of a malic acid salt and a gluconic acid salt. The use of a malic acid salt and gluconic acid salt in combination can mask the sour taste while suppressing bitter and astringent taste. In this embodiment, the substrate more preferably comprises or consists of an alkali metal salt of malic acid and an alkali metal salt of gluconic acid, still more preferably comprises or consists of at least one member selected from the group consisting of sodium malate and potassium malate, and at least one member selected from the group consisting of sodium gluconate and potassium gluconate, and even more preferably comprises or consists of sodium malate and sodium gluconate.

When the substrate comprises a malic acid salt and a gluconic acid salt, the lower limit of the content of the gluconic acid salt is not particularly limited and is, for example, 1 part by mass, preferably 10 parts by mass, and more preferably 20 parts by mass, per 100 parts by mass of the malic acid salt. The upper limit of the content of the gluconic acid salt is not particularly limited and is, for example, 60 parts by mass, 55 parts by mass, or 50 parts by mass, per 100 parts by mass of the malic acid salt.

The substrate (or acetic-acid-containing powder) preferably does not comprise acetic acid salts, such as sodium acetate salt.

The content of the substrate may be, for example, 30 mass% or more, 35 mass% or more, 40 mass% or more, 45 mass% or more, or 50 mass% or more, and may be 80 mass% or less, 75 mass% or less, or 70 mass% or less, based on the acetic-acid-containing powder.

The acetic-acid-containing powder of the present invention may further comprise other components. Examples of the other components include the following components.
- Organic acids other than acetic acid (e.g. malic acid, gluconic acid, citric acid, tartaric acid, succinic acid, and fumaric acid);
- Organic acid salts other than malic acid salts, gluconic acid salts, tartaric acid salts, and lactic acid salts (e.g., citric acid salts, succinic acid salts, and fumaric acid salts);
- Amino acids (e.g., sodium L-glutamate and glycine);
- Nucleic acids (e.g., sodium 5-inosinate and sodium 5-guanylate);
- Sugars (e.g., monosaccharides, such as glucose, disaccharides, such as sucrose, and polysaccharides such as starch, modified starch, dextrin, and CMC);
- Table salt; and
- Food materials (e.g., animal-derived extracts and plant-derived extracts).

These components may be used alone or in a combination of two or more. The content (or total content) of each of these components may be, for example, 20 mass% or less, 15 mass% or less, 10 mass% or less, or 5 mass% or less, and may be 0.01 mass% or more, 0.05 mass% or more, 0.1 mass% or more, 0.5 mass% or more, or 1 mass% or more, based on the acetic-acid-containing powder.

The acidity of the acetic-acid-containing powder of the present invention is not particularly limited. The lower limit of the acidity is, for example, 10%, 15%, 20%, or 25%. The upper limit of the acidity is, for example, 50% or 45%. The acidity of the acetic-acid-containing powder can be determined, for example, by dissolving the acetic-acid-containing powder in water and using HPLC or neutralization titration.

The particle size (or average particle size) of the acetic-acid-containing powder of the present invention can be determined by common methods, such as image analysis, laser diffraction, and centrifugal sedimentation, and may be, for example, 10 um or more, 20 um or more, 30 um or more, 40 um or more, or 50 um or more, and may be 900 um or less, 800 um or less, 700 um or less, 600 um or less, or 500 um or less.

The acetic-acid-containing powder of the present invention can be used, for example, as a food additive. Specifically, like general vinegar, the acetic-acid-containing powder of the present invention can be used for the purpose of adding flavor, such as sour taste, to foods, preserving foods, or increasing the shelf life of foods.

The acetic-acid-containing powder of the present invention can be easily produced, for example, by a method comprising the step of bringing acetic acid or a liquid containing acetic acid into contact (e.g., dropwise addition and/or mixing) with a substrate. In this step, the mixing ratio (mass ratio) of acetic acid to the substrate can be, for example, 5/95 to 60/40, preferably 15/85 to 60/40, and more preferably 25/75 to 60/40.

Acetic acid is typically supplied as a liquid containing acetic acid. Thus, the mass ratio of acetic acid to be mixed to obtain an acetic-acid-containing powder is determined according to the concentration of acetic acid in the liquid. If more than 60 mass% of acetic acid is mixed with the substrate here, the moisture content in the acetic-acid-containing powder will increase, which can cause solidification. Therefore, the acetic acid mixing ratio is preferably 60 mass% or less, whereby the acetic-acid-containing powder can be obtained by reducing the amount of the liquid for dropwise addition, and thus the mixing ratio can be suitably selected.

The present invention encompasses a composition (or a formulation) comprising the acetic-acid-containing powder described above. The composition (or formulation) may be, for example, a seasoning (e.g., acidulant) containing other seasoning ingredients (e.g., sour ingredients), or may be a preservative (or a shelf-life improver) containing an antibacterial ingredient.

The present invention encompasses a food comprising the acetic-acid-containing powder or the composition (or formulation). Examples of foods include, but are not limited to, fresh foods; drinks; processed marine foods, processed meat foods, and salad paste used for home-packed meals and side dishes; noodles, such as pasta and udon; cooked rice; rice cakes; confectioneries; and cooking and seasoning materials.

### Examples

Below, one embodiment of the present disclosure is described in more detail with reference to Examples. However, the present disclosure is not limited thereto.

### Adsorption of Acetic Acid on Various Organic Acid Salts

Fifty parts by weight of glacial acetic acid was added dropwise to 50 parts by weight of sodium malate while stirring. It was confirmed that glacial acetic acid was adsorbed on sodium malate. The same procedure was performed using potassium malate, calcium gluconate, potassium gluconate, potassium tartrate, or calcium lactate instead of sodium malate, and the adsorption of glacial acetic acid was confirmed as in the case in which sodium malate was used.

### Production of Vinegar Powder

### Example 1

Fifty parts by weight of glacial acetic acid was added dropwise to 50 parts by weight of sodium malate while stirring for adsorption, whereby a vinegar powder with fluidity and an acidity of 45% as measured by neutralization titration was obtained.

### Example 2

Fifty parts by weight of glacial acetic acid was added dropwise to 50 parts by weight of sodium malate and 25 parts by weight of sodium gluconate while stirring for adsorption, whereby a vinegar powder with fluidity and an acidity of 36% as measured by neutralization titration was obtained.

### Example 3

Commercially available brewed vinegar containing 20% alcohol by volume was concentrated by freezing the brewed vinegar to -7°C or lower and removing the produced ice crystals. By repeating this operation, the acidity of the brewed vinegar was concentrated to 50%. Fifty parts by weight of the concentrated brewed vinegar was added dropwise to 50 parts by weight of sodium malate while stirring for adsorption, whereby a vinegar powder with fluidity and an acidity of 25% as measured by neutralization titration was obtained.

### Example 4

Commercially available brewed vinegar containing 20% alcohol by volume was frozen and concentrated in the same manner as in Example 3 to obtain brewed vinegar that was concentrated to an acidity of 50%. Fifty parts by weight of the concentrated brewed vinegar was added dropwise to 50 parts by weight of sodium malate and 25 parts by weight of sodium gluconate while stirring for adsorption, whereby a vinegar powder with fluidity and an acidity of 20% as measured by neutralization titration was obtained.

### Example 5

Commercially available brewed vinegar containing 20% alcohol by volume was frozen and concentrated in the same manner as in Example 3 to obtain brewed vinegar that was concentrated to an acidity of 50%. Fifty parts by weight of the concentrated brewed vinegar was added dropwise to 50 parts by weight of potassium gluconate while stirring for adsorption, whereby a vinegar powder with fluidity and an acidity of 25% as measured by neutralization titration was obtained.

### Example 6

Commercially available brewed vinegar containing 20% alcohol by volume was frozen and concentrated in the same manner as in Example 3 to obtain brewed vinegar that was concentrated to an acidity of 50%. Fifty parts by weight of the concentrated brewed vinegar was added dropwise to 50 parts by weight of potassium gluconate and 25 parts by weight of sodium gluconate while stirring for adsorption, whereby a vinegar powder with fluidity and an acidity of 20% as measured by neutralization titration was obtained.

### Comparative Example 1

Fifty parts by weight of glacial acetic acid was added dropwise to 50 parts by weight of anhydrous sodium acetate (produced by Daitou Kagaku KK.) while stirring for adsorption, whereby a wet powder was obtained. The obtained powder was dried with a drying apparatus to obtain a vinegar powder with fluidity and an acidity of 40% as measured by neutralization titration.

### Comparative Example 2

Commercially available brewed vinegar containing 20% alcohol by volume was frozen and concentrated in the same manner as in Example 3 to obtain brewed vinegar that was concentrated to an acidity of 50%. Fifty parts by weight of the concentrated brewed vinegar was added dropwise to 50 parts by weight of anhydrous sodium acetate (produced by Daitou Kagaku KK.) while stirring for adsorption, whereby a wet powder was obtained. The obtained powder was dried with a drying apparatus to obtain a vinegar powder with fluidity and an acidity of 20% as measured by neutralization titration.

### Comparative Example 3

Commercially available brewed vinegar containing 20% alcohol by volume was frozen and concentrated in the same manner as in Example 3 to obtain brewed vinegar that was concentrated to an acidity of 50%. Fifty parts by weight of the concentrated brewed vinegar was added dropwise to 50 parts by weight of dextrin (Max 1000, produced by Matsutani Chemical Industry Co., Ltd.) while stirring. However, removal of only water by drying was impossible due to a too large amount of water contained in the brewed vinegar.

### Sensory Evaluation by Cooked Rice Test for Vinegar Powder Example 1, Example 2, and Comparative Example 1

Example 1, Example 2, or Comparative Example 1 was added to 100 parts by weight of raw rice and 150 parts by weight of water so that the acetic acid content was 0.24%. For a control, none of Example 1, Example 2, or Comparative Example 1 was added. Each mixture was cooked in a home rice cooker to obtain 220 parts by weight of cooked rice.

A sensory evaluation of each of the obtained cooked rice was performed by 12 panelists.

The four test groups, including the non-added group, were rearranged in the order of how strongly bitter and astringent taste was perceived, with 3 points given for the most strongly perceived bitter and astringent taste and 0 points for no perceived bitter and astringent taste, and the average values were obtained.

Next, the same four test groups were rearranged in the order of how strongly sour taste was perceived, with 3 points given for the most strongly perceived sour taste and 0 points for no perceived sour taste, and the average values were obtained.

Table 1 below shows the results of the sensory evaluation of each cooked rice.

**Table 1**

| | Non-added group | Ex. 1 | Ex. 2 | Comp. Ex. 1 |
|---|---|---|---|---|
| Bitter and astringent taste | 0 | 1.9 | 1.2 | 2.9 |
| Sour taste | 0 | 2.1 | 1.2 | 2.7 |

### Example 3, Example 4, and Comparative Example 2

Example 3, Example 4, or Comparative Example 2 was added to 100 parts by weight of raw rice and 150 parts by weight of water so that the acetic acid content was 0.24%. For a control, none of Example 3, Example 4, or Comparative Example 2 was added. Each mixture was cooked in a home rice cooker to obtain 220 parts by weight of cooked rice.

A sensory evaluation of each of the obtained cooked rice was performed by 12 panelists.

The four test groups, including the non-added group, were rearranged in the order of how strongly bitter and astringent taste was perceived, with 3 points given for the most strongly perceived bitter and astringent taste and 0 points for no perceived bitter and astringent taste, and the average values were obtained.

Next, the same four test groups were rearranged in the order of how strongly sour taste was perceived, with 3 points given for the most strongly perceived sour taste and 0 points for no perceived sour taste, and the average values were obtained.

Table 2 below shows the results of the sensory evaluation of each cooked rice.

**Table 2**

| | Non-added group | Ex. 3 | Ex. 4 | Comp. Ex. 2 |
|---|---|---|---|---|
| Bitter and astringent taste | 0 | 1.9 | 1.3 | 2.8 |
| Sour taste | 0 | 2.0 | 1.2 | 2.8 |

### Example 5, Example 6, and Comparative Example 2

Example 5, Example 6, or Comparative Example 2 was added to 100 parts by weight of raw rice and 150 parts by weight of water so that the acetic acid content was 0.24%. For a control, none of Example 5, Example 6, or Comparative Example 2 was added. Each mixture was cooked in a home rice cooker to obtain 220 parts by weight of cooked rice.

A sensory evaluation of each of the obtained cooked rice was performed by 12 panelists.

The four test groups, including the non-added group, were rearranged in the order of how strongly bitter and astringent taste was perceived, with 3 points given for the most strongly perceived bitter and astringent taste and 0 points for no perceived bitter and astringent taste, and the average values were obtained.

Next, the same four test groups were rearranged in the order of how strongly sour taste was perceived, with 3 points given for the most strongly perceived sour taste and 0 points for no perceived sour taste, and the average values were obtained.

Table 3 below shows the results of the sensory evaluation of each cooked rice.

**Table 3**

| | Non-added group | Ex. 5 | Ex. 6 | Comp. Ex. 2 |
|---|---|---|---|---|
| Bitter and astringent taste | 0 | 2.1 | 1.4 | 2.8 |
| Sour taste | 0 | 2.3 | 1.5 | 2.9 |

## Claims

1. An acetic-acid-containing powder comprising acetic acid or a liquid containing acetic acid, and a substrate, wherein the acetic acid or the liquid containing acetic acid is adsorbed on the substrate, the substrate comprising at least one member selected from the group consisting of malic acid salts, gluconic acid salts, tartaric acid salts, and lactic acid salts.

2. The acetic-acid-containing powder according to claim 1, wherein the substrate comprises at least one member selected from the group consisting of sodium malate, potassium malate, sodium gluconate, potassium gluconate, calcium gluconate, potassium tartrate, and calcium lactate.
